# EUROPÄISCHE PATENTSCHRIFT

(11) **EP 4 214 479 B1**
(45) Veröffentlichungstag und Bekanntmachung des Hinweises auf die Patenterteilung: **02.10.2024**
(21) Anmeldenummer: 21770179.6
(22) Anmeldetag: 30.08.2021
(51) Int. Cl.: G01F 23/00, G01F 23/26, G01N 24/00, G01R 33/12, G01R 33/16, G01R 33/26, G01R 33/32, G01F 23/284, G01F 23/292, G01R 33/032

(54) **QUANTENSENSOR**
QUANTUM SENSOR
CAPTEUR QUANTIQUE

(30) Priorität: 15.09.2020 DE 102020123993
(43) Veröffentlichungstag der Anmeldung: 26.07.2023
(73) Patentinhaber: Endress+Hauser SE+Co. KG, 79689 Maulburg (DE)
(72) Erfinder: FRÜHAUF, Dietmar, 79539 Lörrach (DE); THAM, Anh Tuan, 14089 Berlin (DE); EBRAHIMI, Mohammad Sadegh, 79539 Lörrach (DE); BECHTEL, Gerd, 79585 Steinen (DE); KUHNEN, Raphael, 79418 Schliengen (DE); BRENGARTNER, Tobias, 79312 Emmendingen (DE)
(74) Vertreter: Hahn, Christian
(86) Internationale Anmeldenummer: PCT/EP2021/073825
(87) Internationale Veröffentlichungsnummer: WO 2022/058141

(56) Entgegenhaltungen:
- WO-A1-2020/060694
- DE-A1- 102014 219 547
- DE-A1- 102014 219 550
- DE-A1- 102017 205 099
- DE-A1- 102017 206 279
- DE-A1- 3 341 265
- DE-A1- 3 742 878
- DE-U1- 202017 100 802
- JP-A- 2015 034 706
- JP-B2- 3 427 801
- US-A- 4 695 796
- US-A- 5 321 258

## Beschreibung

Die vorliegende Erfindung betrifft eine Sensorvorrichtung zur Bestimmung und/oder Überwachung einer Prozessgröße eines Mediums in einem Behältnis sowie ein Verfahren zur Bestimmung und/oder Überwachung der Prozessgröße mittels einer erfindungsgemäßen Sensorvorrichtung.

Feldgeräte zur Überwachung und/oder Bestimmung mindestens einer, beispielsweise chemischen oder physikalischen, Prozessgröße eines Mediums sind in unterschiedlichsten Ausgestaltungen aus dem Stand der Technik bekannt. Im Rahmen der vorliegenden Anmeldung werden im Prinzip alle Messgeräte als Feldgerät bezeichnet werden, die prozessnah eingesetzt werden und die prozessrelevante Informationen liefern oder verarbeiten, also auch Remote I/Os, Funkadapter bzw. allgemein elektronische Komponenten, die auf der Feldebene angeordnet sind. Eine Vielzahl solcher Feldgeräte wird von Firmen der Endress + Hauser-Gruppe hergestellt und vertrieben.

Eine neuere Entwicklung im Bereich der Sensorik stellen sogenannte Quantensensoren dar, bei welchen unterschiedlichste Quanteneffekte zur Bestimmung verschiedener physikalischer und/oder chemischer Messgrößen ausgenutzt werden. Beispielsweise sind solche Ansätze im Bereich der industriellen Prozessautomatisierung insbesondere mit Hinblick auf ein zunehmendes Bestreben zur Miniaturisierung bei gleichzeitiger Steigerung der Leistungsfähigkeit der jeweiligen Sensoren interessant.

De DE3742878A1 beschreibt beispielsweise einen optischen Magnetfeldsensor, indem ein Kristall als magnetempfindliches optisches Bauteil verwendet wird.

Aus der DE102017205099A1 ist eine Sensorvorrichtung mit einem Kristallkörper mit zumindest einer Fehlstelle, einer Lichtquelle, einer Hochfrequenzeinrichtung zum Beaufschlagen des Kristallkörpers mit einem Hochfrequenzsignal, und einer Detektionseinheit zur Detektion einer magnetfeldabhängigen Fluoreszenzsignals bekannt geworden. Die Lichtquelle ist auf einem ersten Substrat und die Detektionseinrichtung auf einem zweiten Substrat angeordnet, während die Hochfrequenzeinrichtung und der Kristallkörper auf beiden, miteinander verbundenen Subtraten angeordnet sein können. Als Messgrößen kommen externe Magnetfelder, elektrische Ströme, eine Temperatur, mechanische Spannungen oder ein Druck in Frage. Ausführlich diskutiert wird die Anwendung der Sensorvorrichtung zur Magnetfelderfassung, um damit eine berührungslose Strommessung zu realisieren. Durch ein zusätzliches Referenzmagnetfeld ist eine Kalibrierung der Sensorvorrichtung möglich. Eine ähnliche Vorrichtung ist aus der DE102017205265A1 bekannt geworden.

Die DE102014219550A1 beschreibt einen Kombinationssensor zur Erfassung von Druck, Temperatur und/oder Magnetfeldern, wobei das Sensorelement eine Diamantstruktur mit zumindest einem Stickstoff-Vakanz-Zentrum aufweist.

Die DE102018214617A1 offenbart eine Sensoreinrichtung, welche ebenfalls einen Kristallkörper mit einer Anzahl von Farbzentren, bei welcher zur Steigerung der Effektivität und zur Miniaturisierung verschiedene optische Filterelemente verwendet werden.

In der DE102016210259A1 wird eine weitere Ausgestaltung für eine Sensorvorrichtung sowie eine Kalibrations- und Auswertemethode basierend auf Fehlstellen in einem Kristall vorgeschlagen. Die Sensorvorrichtung umfasst einen Kristallkörper mit zumindest einer Fehlstelle, eine Lichtquelle, eine Mikrowellenantenne zur Beaufschlagung des Kristallkörpers mit Mikrowellen, eine Detektionseinrichtung zur Erfassung einer Fluoreszenz von dem Kristallkörper, und eine Anlegeeinrichtung, mittels welcher ein Induktionsstrom an die Mikrowellenantenne anlegbar ist. So dient die Mikrowellenantenne zum einen zur Erzeugung der Mikrowellen und zur Erzeugung eines internen Magnetfelds. Das interne Magnetfeld ermöglicht eine Kalibrierung im fortlaufenden Betrieb.

Die DE 10 2014 219 547 A1 offenbart einen Drucksensor mit einer drucksensitiven Schicht, die durch Fluoreszenzsignale ausgelesen wird.

Der WO 2020/060694 A1 lässt sich entnehmen, dass ein Sensor für eine berührungslose Füllstandsmessung eines Mediums in einem Behälter an die Außenseite des Behälters angebracht wird.

Die US 5,321,258 A offenbart die Verwendung eines Lichtleiters für einen optischen Sensor.

Ausgehend vom Stand der Technik liegt der vorliegenden Erfindung die Aufgabe zugrunde, den Anwendungsbereich derartiger Technologien zu erweitern.

Diese Aufgabe wird gelöst durch eine Sensorvorrichtung sowie durch ein Verfahren.

Hinsichtlich der Sensorvorrichtung wird die der Erfindung zugrunde liegende Aufgabe gelöst durch eine Sensorvorrichtung zur Bestimmung und/oder Überwachung einer Prozessgröße eines Mediums in einem Behältnis, umfassend
- einen Kristallkörper mit zumindest einer Fehlstelle,
- eine Magnetfeld-Einrichtung zur Erzeugung eines Magnetfelds,
   wobei die Magnetfeld-Einrichtung derart angeordnet ist, dass mittels der Magnetfeld-Einrichtung ein Magnetfeld im Bereich des Kristallkörpers und im Bereich des sich innerhalb des Behältnisses befindenden Mediums erzeugbar ist, und
   wobei der Kristallkörper und die Magnetfeld-Einrichtung von außen an einer Wandung des Behältnisses anordenbar sind,
- eine Detektionseinheit zur Detektion eines magnetfeldabhängigen, Fluoreszenzsignals von dem Kristallkörper,
   wobei die Detektionseinheit eine Anregeeinheit zur optischen Anregung der Fehlstelle und einen Detektor zur Detektion des Fluoreszenzsignals aufweist,
   und
- eine Auswerte-Einheit zum Ermitteln zumindest einer Aussage über die Prozessgröße anhand des Fluoreszenzsignals.

Die Fehlstellen in dem Kristallkörper werden optisch angeregt und die Fluoreszenz der Fehlstellen detektiert und ausgewertet. Zusätzlich wird mittels der Magnetfeld-Einrichtung ein Magnetfeld im Bereich des Kristallkörpers und im Bereich des Behältnisses erzeugt. Dieses Magnetfeld wird wiederum durch im Behälter vorhandene Medium beeinflusst, beispielsweise durch An- oder Abwesenheit des Mediums, eine Änderung des Füllstands, oder die Änderung zumindest einer, insbesondere physikalischen oder chemischen Eigenschaft des Mediums. Die Änderung des Magnetfelds wiederum beeinflusst das Fluoreszenzsignal der Fehlstellen, so dass anhand des magnetfeldabhängigen Fluoreszenzsignals eine Prozessüberwachung möglich wird bzw. eine Prozessgröße ermittelbar ist. Vorteilhaft lassen sich anhand von Fehlstellen in Kristallkörpern sehr hohe Messauflösungen hinsichtlich der medienbedingten Änderung des Magnetfelds erfassen. Dies erlaubt eine sehr hohe Messgenauigkeit.

Zudem handelt es sich bei der erfindungsgemäßen Sensorvorrichtung um eine frontbündig mit der Wandung des Behältnisses abschließende oder eine nicht invasive, von außen an dem Behältnis befestigbare Vorrichtung. Eine Bestimmung einer oder mehrerer Prozessgrößen mittels einer frontbündigen oder nicht invasiven Messanordnung ist im Bereich der Prozessautomatisierung aus unterschiedlichen Gründen sehr vorteilhaft und es besteht ein allgemeines Bestreben, derartige Lösungen für unterschiedlichste Anwendungen zu realisieren, was jedoch im Einzelfall schwierig sein kann. So sind nur vereinzelt derartige Geräte verfügbar, welche in ihrem Anwendungsspektrum jeweils begrenzt sind. Mit der vorliegenden Erfindung wird nun ein frontbündiger oder nicht-invasiver Sensor bereitgestellt, welcher vielfältige Anwendungen erlaubt und mittels welchem eine umfassende Prozessanalyse möglich ist.

Bei dem Behältnis handelt es sich insbesondere um einen Behälter oder eine Rohrleitung.

Die Detektionseinheit umfasst Anregeeinheit zur optischen Anregung der Fehlstelle, welche beispielsweise ein Laser oder eine Leuchtdiode (LED) sein kann. Bei dem Detektor wiederum kann es sich beispielsweise um einen Photodetektor oder einen CMOS-Sensor handeln. Zudem kann die Detektionseinheit über weitere optische Elemente verfügen, wie beispielsweise verschiedene Filter, Linsen oder Spiegel.

Gemäß einer Ausgestaltung handelt es sich bei dem Kristallkörper um einen Diamanten mit zumindest einer Stickstoff-Fehlstelle, um Siliziumcarbid mit zumindest einer Silizium-Fehlstelle oder um hexagonales Bornitrid mit zumindest einem Fehlstellen-Farbzentrum.

Der Kristallkörper umfasst erfindungsgemäß zumindest eine Fehlstelle. Es können jedoch auch mehrere Fehlstellen zum Einsatz kommen. In diesem Falle ist insbesondere eine lineare Anordnung der Fehlstellen bevorzugt. Mehrere Fehlstellen führen zu einer erhöhten Intensität, so dass die Messauflösung verbessert bzw. Intensitätsänderungen auch bei vergleichsweise schwachen Magnetfeldern detektierbar sind.

Gemäß einer weiteren Ausgestaltung umfasst die Magnetfeld-Einrichtung zumindest eine Spule. In diesem Zusammenhang ist es von Vorteil, wenn die Spule den Kristallkörper zumindest teilweise umgibt, wenn der Kristallkörper und die Magnetfeld-Einrichtung an der Wandung des Behältnisses angeordnet sind. Der Kristallkörper ist vorzugsweise zumindest teilweise innerhalb der Spule angeordnet.

Einerseits ist eine Kompaktbauweise der Sensorvorrichtung denkbar. In diesem Falle ist die Detektionseinheit in unmittelbarer Umgebung zur Magnetfeld-Einrichtung und zu dem Kristallkörper angeordnet. In einer anderen Ausgestaltung umfasst die Sensorvorrichtung jedoch zudem eine Lichtleitfaser zur Leitung des Fluoreszenzsignals von dem Kristallkörper zur Detektionseinheit. In letzterem Fall ist die Detektionseinheit von den anderen Komponenten der Sensorvorrichtung räumlich getrennt angeordnet. Welche Variante jeweils zu bevorzugen ist, hängt von der konkreten Anwendung der Sensorvorrichtung ab. Die Messgenauigkeit hängt dabei unter anderem entscheidend von den Komponenten der Detektionseinheit, beispielsweise der verwendeten Anregeeinheit, den optischen Komponenten usw., ab. Die Detektionseinheit sowie ihre Anordnung relativ zu den restlichen Komponenten der Sensorvorrichtungen werden entsprechend in Abhängigkeit der angedachten Verwendung gewählt.

Zudem ist es einerseits denkbar, die Sensorvorrichtung von außen an der Wandung des Behältnisses zu befestigen. In diesem Falle kann eine nicht-invasive Bestimmung der zumindest einen Prozessgröße erfolgen. Eine alternative Ausgestaltung beinhaltet jedoch, dass die Sensorvorrichtung derart ausgestaltet ist, dass sie frontbündig in die Wandung des Behältnisses einbringbar ist. So umfasst die Sensorvorrichtung in einer Ausgestaltung eine Einfassung zur Einbringung zumindest einer Komponente der Sensorvorrichtung in eine Wandung des Behältnisses. Bei dieser Einfassung kann es sich um einen Ausschnitt der Wandung des Behältnisses handeln, welcher in eine korrespondierend ausgestaltete Öffnung in der Wandung des Behältnisses einbringbar ist. Die Einfassung ist aus einem vorgebbaren Material, beispielsweise rostfreiem Edelstahl, hergestellt. Ein Vorteil in diesem Zusammenhang besteht darin, dass mittels der Einfassung Einflüsse der Wandung des Behältnisses auf die Bestimmung der jeweiligen Prozessgröße minimiert oder eliminiert werden können.

In weiteren Ausgestaltungen der erfindungsgemäßen Sensorvorrichtung kann zudem eine Einheit zur Anregung von Hochfrequenz- oder Mikrowellenstrahlung vorhanden. Dies ermöglicht die Anregung von Elektronen in höhere Energieniveaus.

Die der Erfindung zugrunde liegende Aufgabe wird ferner gelöst durch ein Verfahren zur Bestimmung und/oder Überwachung einer Prozessgröße eines Mediums in einem Behältnis mittels einer erfindungsgemäßen Sensorvorrichtung, umfassend folgende Verfahrensschritte:
- Erzeugen eines Magnetfelds im Bereich des Kristallkörpers und im Bereich des sich innerhalb des Behältnisses befindenden Mediums,
- Anregen der Fehlstelle in dem Kristallkörper zur Fluoreszenz,
- Detektieren des magnetfeldabhängigen, Fluoreszenzsignals von dem Kristallkörper, und
- Ermitteln zumindest einer Aussage über die Prozessgröße anhand des Fluoreszenzsignals.

Vorteilhaft zeichnen sich das erfindungsgemäße Verfahren sowie die erfindungsgemäße Sensoranordnung durch eine sehr hohe Messgenauigkeit, sowohl bezüglicher absoluter als auch relativer Aussagen über das jeweilige Medium, aus. Bereits kleinste Änderungen des Magnetfelds, welche durch das Medium oder Änderungen im Bereich des Mediums hervorgerufen werden, sind mit hoher Präzision detektierbar, die mit herkömmlichen Messgeräten gemäß Stand der Technik nicht erreichbar sind.

Eine Ausgestaltung des Verfahrens beinhaltet, dass anhand des Fluoreszenzsignals zumindest eine für das Magnetfeld charakteristische Größe, insbesondere die magnetische Suszeptibilität oder die magnetische Permeabilität, ermittelt wird. Diese Magnetfeld-Kenngröße wiederum kann zur Ermittlung der Aussage über die Prozessgröße herangezogen werden.

In diesem Zusammenhang ist es beispielsweise von Vorteil, wenn anhand der für das Magnetfeld charakteristischen Größe zumindest eine physikalische und/oder chemische Kenngröße des Mediums ermittelt wird. Hierbei kann es sich beispielsweise um die magnetische Permeabilität des Mediums, insbesondere im Falle von nicht elektrisch leitfähigen Medien, handeln. Im Falle von elektrisch leitfähigen Medien ist jedoch beispielsweise auch die elektrische Leitfähigkeit ermittelbar, beispielsweise anhand eines Wirbelstroms. Aber auch andere Qualitätsparameter des Mediums können bestimmt werden.

Es ist ebenfalls von Vorteil, wenn anhand der für das Magnetfeld charakteristischen Größe eine Zustandsüberwachung eines in dem Behältnis ablaufenden Prozesses gemacht wird. Insbesondere kann die für das Magnetfeld charakteristische Größe, oder eine davon abgeleitete Größe als Funktion der Zeit betrachtet werden. Als Beispiele für mögliche Zustandsüberwachungen kommen beispielsweise Mischvorgänge von zumindest zwei Medien oder die Überwachung innerhalb des Behälters ablaufender chemischer Reaktionen in Frage.

Die erfindungsgemäße Sensorvorrichtung bzw. das erfindungsgemäße Verfahren ermöglichen somit eine umfangreiche Prozessüberwachung.

Eine weitere Ausgestaltung des erfindungsgemäßen Verfahrens beinhaltet, dass ein vorgebbarer Grenzstand des Mediums in dem Behältnis überwacht wird. Die erfindungsgemäße Sensoreinrichtung kann somit auch als Grenzstandschalter zur Überwachung eines vorgebbaren Füllstands des Mediums in dem Behältnis verwendet werden.

In diesem Zusammenhang ist es von Vorteil, wenn ein Grenzwert für die für das Magnetfeld charakteristischen Größe vorgegeben wird, und wobei im Falle eines Über- oder Unterschreitens des Grenzwerts ein Erreichen des vorgebbaren Grenzstands signalisiert wird.

Es ist im Zusammenhang mit dem erfindungsgemäßen Verfahren sowohl denkbar, ein Wechselfeld als auch ein Gleichfeld zu erzeugen. Während Wechselfelder insbesondere mit Hinblick auf die Auswertung der Messsignale vorteilhaft sind, ergeben sich im Falle leitfähiger Behältnisse Einflüsse durch sich ausbildende Wirbelströme, welche bei der Auswertung zu berücksichtigen sind. In diesem Zusammenhang kann eine frontbündige Ausgestaltung der Sensorvorrichtung mit einer geeigneten Einfassung aus bekanntem Material von Vorteil sein, da auf diese Weise der Einfluss der entstehenden Wirbelströme bekannt ist und minimiert oder eliminiert werden kann.

Schließlich ist es in einer Ausgestaltung des erfindungsgemäßen Verfahrens vorgesehen, dass im Bereich des Kristallkörpers und im Bereich des sich innerhalb des Behältnisses befindenden Mediums erzeugte Magnetfeld in Form eines Wechselfelds zu erzeugen, und die Frequenz des Wechselfelds zu variieren. Die Eindringtiefe des Magnetfelds durch eine Wandung des Behältnisses, insbesondere im Falle von leitfähigen Behältnissen bzw. die Eindringtiefe in das Medium hinein ist abhängig von der Frequenz des Magnetfelds. Die Eindringtiefe nimmt mit abnehmender Frequenz zu. Indem die Frequenz des Wechselfelds variiert wird, kann eine mehrdimensionale Analyse des Mediums in dem Behältnis vorgenommen werden. So können beispielsweise Ansätze, Gradienten bezüglich der Zusammensetzung des Mediums in Abhängigkeit vom Ort innerhalb des Behältnisses oder auch Ablagerungen erkannt werden.

Es sei darauf verwiesen, dass die im Zusammenhang mit der erfindungsgemäßen Sensorvorrichtung beschriebenen Ausgestaltungen mutatis mutandis auf das erfindungsgemäße Verfahren anwendbar sind und umgekehrt.

Die Erfindung wird anhand der nachfolgenden Figuren näher erläutert. Es zeigt:
Fig. 1 ein vereinfachtes Energieschema für negative NV-Zentren in Diamant,
Fig. 2 eine erste Ausgestaltung einer erfindungsgemäßen Sensorvorrichtung, und
Fig. 3 eine zweite Ausgestaltung einer erfindungsgemäßen Sensorvorrichtung.

In den Figuren sind gleiche Elemente mit demselben Bezugszeichen versehen.

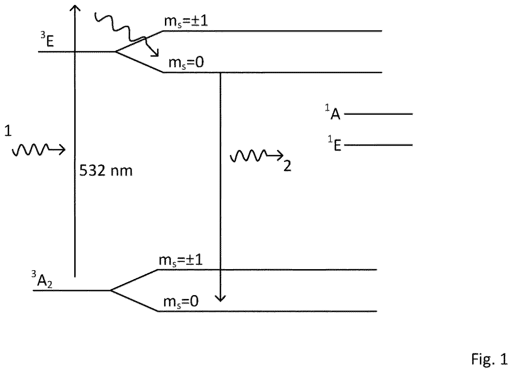

Anhand von Fig. 1 wird zunächst die Anregung einer Fluoreszenz einer Fehlstelle in einem Kristallkörper beispielhaft für den Fall eines NV-Zentrums in Diamant erläutert. Die Überlegungen lassen sich auf andere Kristallkörper mit entsprechenden Fehlstellen übertragen.

Diamant als eine der elementaren Kohlenstoffmodifikationen weist eine kubisch flächenzentrierte Kristallstruktur mit zweiatomiger Basis. Als NV-Zentrum bezeichnet man ein fehlendes Kohlenstoffatom (V) auf einem Gitterplatz mit einem Stickstoffatom (N) als einem der vier nächsten Nachbarn. Für die Anregung und Auswertung von Fluoreszenzsignalen sind insbesondere die negativen NV⁻ -Zentren von Bedeutung, welchen ein zusätzliches Elektron aus dem Diamantgitter zugeordnet ist.

Diamantstruktur mit eingebetteten, negativen NV-Zentren sind der Symmetriegruppe C₃ᵥ zugeordnet, welche die möglichen räumlichen Eigenzustände des NV-Zentrums vorgibt. Wie anhand des Energie-Schemas ohne resonante Anregung und ohne externes Magnetfeld in Fig. 1 ersichtlich, ergibt sich ein Triplet-Grundzustand ³A₂ und ein angeregter Triplett-Zustand ³E, zwischen welchen zwei metastabile Singulett-Zustände ¹E und ¹A liegen. Der Triplet-Grundzustand ³A₂ besitzt drei magnetische Unterzustände mₛ=0,±1 und auch beim angeregten ³E-Zustand gibt es eine energetische Aufspaltung.

Durch Anregung 1 mit Licht der Wellenlänge λ=532 nm kommt es beispielsweise zu einer Anregung eines Vibrationszustands des angeregten 3E-Zustands mit anschließendem Frank-Condon Übergang zum Grundzustand ³A₂, bei welchem ein Fluoreszenz-Photon 2 mit einer Wellenlänge von λ=630 nm ausgesandt wird. Im Falle des Anlegens eines externen Magnetfelds kommt es zudem zu einem Zeeman-Splitting der Energieniveaus und damit einhergehend zu zwei Fluoreszenz-Minima, deren Abstand beispielsweise proportional zur angelegten Magnetfeldstärke B ist.

Die Auswertung des Fluoreszenzlichts kann im Rahmen der vorliegenden Erfindung jedoch auf viele unterschiedliche Arten und Weisen erfolgen. Neben der zuvor skizzierten Auswertung der Energiedifferenz zwischen den beiden Energieniveaus, welche eine Ermittlung des Magnetfelds anhand der Zeeman-Formel ermittelt wird, kann in einer alternativen optischen Auswertungsmethode auch die Intensität des ausgestrahlten Lichts betrachtet werden, die ebenfalls dem Magnetfeld proportional ist. Eine elektrische Auswertung wiederum kann beispielsweise über eine Photocurrent Detection of Magnetic Resonance (engl. kurz PDMR) erfolgen. Neben diesen Beispielen zur Auswertung des Fluoreszenzsignals sind noch weitere Möglichkeiten vorhanden, welche ebenfalls unter die vorliegende Erfindung fallen.

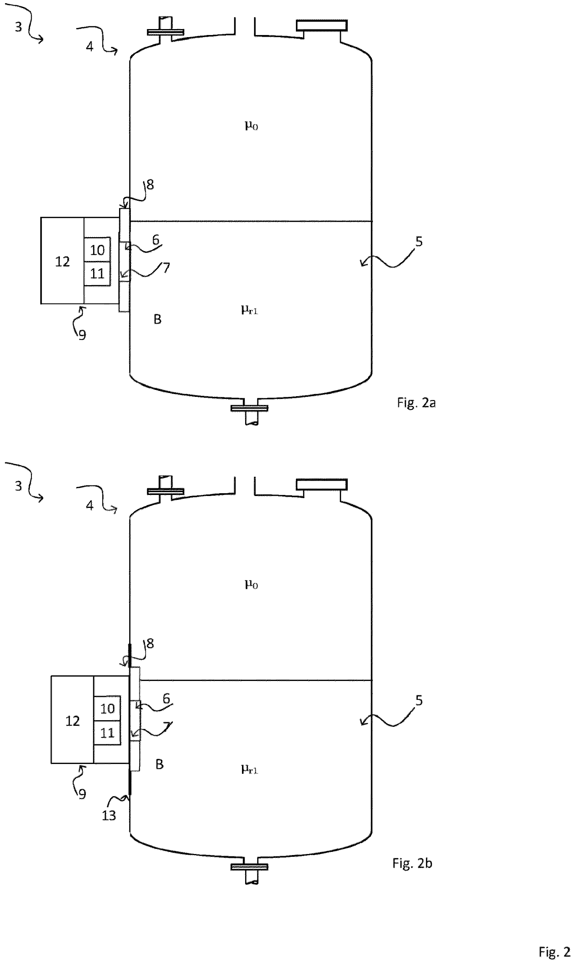

In Fig. 2 sind erste mögliche und exemplarische Ausgestaltungen einer erfindungsgemäßen Sensorvorrichtung 3 dargestellt, welche an einer äußeren Wandung eines Behältnisses 4 in Form eines Behälters, der teilweise mit dem Medium 5 gefüllt ist, angebracht ist. Die Sensorvorrichtung 3 umfasst einen Kristallkörper 6 mit zumindest einer Fehlstelle 7, und eine Magnetfeld-Einrichtung 8 mit zumindest einer Spule, welche den Kristallkörper 6 zumindest teilweise umgibt. Für die hier gezeigte Ausgestaltung ist der Kristallkörper 6 innerhalb der Spule 8 angeordnet. Die Magnetfeld-Einrichtung 8 dient zur Erzeugung eines Magnetfelds B im Bereich des Kristallkörpers 6 und im Bereich eines Innenvolumens des Behältnisses 4.

Zudem umfasst die Sensorvorrichtung eine Detektionseinheit 9 mit einer Anregeeinheit 10 und einem Detektor 11 zur Detektion des Fluoreszenzsignals 2, sowie eine Auswerte-Einheit 12 zur weiteren Auswertung des Signals 2 und zur Ermittlung der zumindest einen Aussage über die Prozessgröße.

Während in Fig. 2a eine nicht-invasive Sensorvorrichtung 3 dargestellt ist, welche von außen an der Wandung des Behältnisses 4 befestigbar ist, ist in Fig. 2b eine frontbündig mit der Wandung des Behälters 4 abschließende Vorrichtung 3 dargestellt, welche eine Einfassung 13 aufweist, mittels welcher zumindest eine Komponente der Sensorvorrichtung 3, hier der Kristallkörper 6 und die Spule, in einer Öffnung der Wandung des Behälters 4 befestigbar ist.

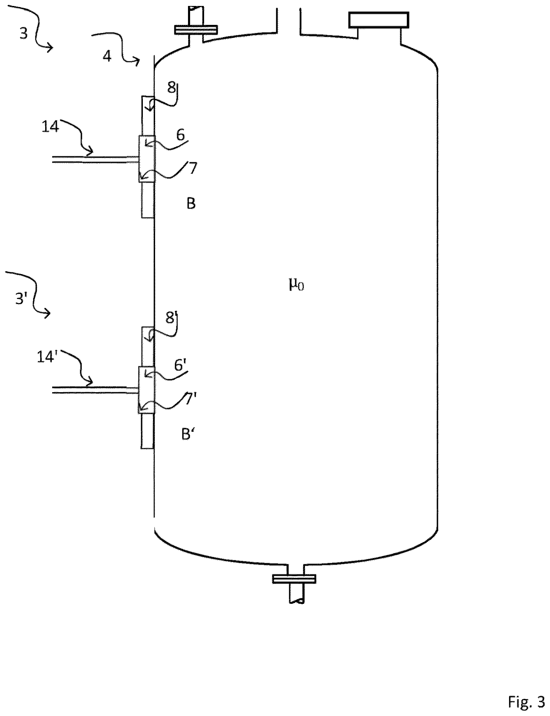

In Fig. 3 ist eine weitere mögliche Ausgestaltung eines Systems mit zwei erfindungsgemäßen Vorrichtungen 3 und 3` dargestellt. Im Unterschied zu der Ausgestaltung aus Fig. 2 ist im Falle der Fig. 3 jeweils keine Kompaktbauweise gewählt, sondern die Detektionseinheit 9, 9` und die Auswerte-Einheit 12, 12' sind räumlich von den restlichen Komponenten der Sensorvorrichtungen 3 und 3` angeordnet und hier deshalb nicht dargestellt. Um jeweils das Fluoreszenzsignal 2 zur Detektionseinheit 9, 9` leiten zu können, umfassen die beiden gezeigten Sensorvorrichtungen 3, 3` jeweils eine Lichtleitfaser 14, 14' zur Leitung des Lichts von dem Kristallkörper 6, 6` zur Detektionseinheit 9, 9`. Es ist sowohl denkbar, eine Detektionseinheit 9 für beide Kristallkörper 6, als auch zwei getrennte Detektionseinheiten 9, 9' zu verwenden. Die Anregeeinheit 11 kann im Bereich des Behälters 4 oder zusammen mit der Auswerte-Einheit 12 angeordnet sein. Auch für die Anregeeinheit 11 ist es sowohl möglich, zwei getrennte oder eine gemeinsame Anregeeinheit 11 zu verwenden.

Die vorliegende Sensorvorrichtung 3 und das erfindungsgemäße Verfahren erlauben einer umfassende Prozessüberwachung, welche die Möglichkeiten mehrerer möglicher gängiger Messverfahren, welche aus dem Stand der Technik um Gebiet der Prozessautomatisierung bekannt sind. Es sind zum einen Prozessgrößen wie ein vorgebbarer Füllstand des Mediums in dem Behältnis überwachbar. Darüber hinaus kann aber auch eine umfassende Charakterisierung des Mediums 5 bzw. von innerhalb des Behältnisses 4 ablaufender Prozesse durchgeführt werden. Zudem handelt es sich bei der erfindungsgemäßen Vorrichtung 3 vorteilhaft um einen nicht-invasiven Sensor, welcher also keinen Eingriff in den fortlaufenden Prozess erforderlich macht und welcher auf einfache Art und Weise eine Miniaturisierung der jeweiligen Sensorvorrichtung bei gleichzeitig breitem Einsatzgebiet ermöglicht.

### Bezugszeichen

- 1: Anregungslicht
- 2: Fluoreszenzlicht
- 3: Sensorvorrichtung
- 4: Behältnis
- 5: Medium
- 6: Kristallkörper
- 7: Fehlstelle
- 8: Magnetfeld-Einrichtung
- 9: Detektionseinheit
- 10: Anrege-Einheit
- 11: Detektor
- 12: Auswerte-Einheit
- 13: Einfassung
- 14: Lichtleitfaser

## Patentansprüche

1. Sensorvorrichtung (3) zur Bestimmung und/oder Überwachung einer Prozessgröße eines Mediums (5) in einem Behältnis (4),
umfassend
- einen Kristallkörper (6) mit zumindest einer Fehlstelle (7),
- eine Magnetfeld-Einrichtung (8) zur Erzeugung eines Magnetfelds,
wobei die Magnetfeld-Einrichtung (8) derart angeordnet ist, dass mittels der Magnetfeld-Einrichtung (8) ein Magnetfeld (B) im Bereich des Kristallkörpers (6) und im Bereich des sich innerhalb des Behältnisses (4) befindenden Mediums (5) erzeugbar ist, und
wobei der Kristallkörper (6) und die Magnetfeld-Einrichtung (8) von außen an einer Wandung des Behältnisses (4) anordenbar sind,
- eine Detektionseinheit (9) zur Detektion eines magnetfeldabhängigen Fluoreszenzsignals (2) von dem Kristallkörper (6),
wobei die Detektionseinheit (9) eine Anregeeinheit (10) zur optischen Anregung der Fehlstelle (7) und einen Detektor (11) zur Detektion des Fluoreszenzsignals (2) aufweist, und
- eine Auswerte-Einheit (12) zum Ermitteln zumindest einer Aussage über die Prozessgröße anhand des Fluoreszenzsignals (2).

2. Sensorvorrichtung (3) nach Anspruch 1,
wobei die Magnetfeld-Einrichtung (8) zumindest eine Spule umfasst, und
wobei die Spule den Kristallkörper (6) zumindest teilweise umgibt, wenn der Kristallkörper (6) und die Magnetfeld-Einrichtung (8) an der Wandung des Behältnisses (4) angeordnet sind.

3. Sensorvorrichtung (3) nach Anspruch 1 oder 2,
umfassend eine Lichtleitfaser (14) zur Leitung des Fluoreszenzsignals (2) von dem Kristallkörper (6) zur Detektionseinheit (9).

4. Sensorvorrichtung (3) nach einem der Ansprüche 1 bis 3,
umfassend eine Einfassung (13) zur Einbringung zumindest einer Komponente (6) der Sensorvorrichtung (3) in eine Wandung des Behältnisses (4).

5. Verfahren zur Bestimmung und/oder Überwachung einer Prozessgröße eines Mediums (5) in einem Behältnis (4) mittels einer Sensorvorrichtung (3) nach einem der vorherigen Ansprüche 1 bis 4,
umfassend folgende Verfahrensschritte:
- Erzeugen eines Magnetfelds (B) im Bereich des Kristallkörpers (6) und im Bereich des sich innerhalb des Behältnisses (4) befindenden Mediums (5),
- Anregen der Fehlstelle (7) in dem Kristallkörper (6) zur Fluoreszenz,
- Detektieren des magnetfeldabhängigen Fluoreszenzsignals (2) von dem Kristallkörper (6), und
- Ermitteln der zumindest einen Aussage über die Prozessgröße anhand des Fluoreszenzsignals (2).

6. Verfahren nach Anspruch 5,
wobei anhand des Fluoreszenzsignals (2) zumindest eine für das Magnetfeld (B) charakteristische Größe, insbesondere die magnetische Suszeptibilität oder die magnetische Permeabilität, ermittelt wird.

7. Verfahren nach Anspruch 6,
wobei anhand der für das Magnetfeld (B) charakteristischen Größe zumindest eine physikalische und/oder chemische Kenngröße des Mediums (5) ermittelt wird.

8. Verfahren nach Anspruch 6 oder 7,
wobei anhand der für das Magnetfeld (B) charakteristischen Größe eine Zustandsüberwachung eines in dem Behältnis (4) ablaufenden Prozesses gemacht wird.

9. Verfahren nach einem der Ansprüche 6 bis 8,
wobei ein vorgebbarer Grenzstand des Mediums (5) in dem Behältnis (4) überwacht wird, wobei ein Grenzwert für die für das Magnetfeld (B) charakteristischen Größe vorgegeben wird, und
wobei im Falle eines Über- oder Unterschreitens des für das Magnetfeld (B) charakteristischen Größe vorgegebenen Grenzwerts ein Erreichen des vorgebbaren Grenzstands des Mediums (5) signalisiert wird.

10. Verfahren nach einem der Ansprüche 6 bis 9,
wobei das im Bereich des Kristallkörpers (6) und im Bereich des sich innerhalb des Behältnisses (4) befindenden Mediums (5) erzeugte Magnetfeld (B) in Form eines Wechselfelds erzeugt wird, und
wobei die Frequenz des Wechselfelds (B) variiert wird.

## Claims

1. Sensor device (3) for determining and/or monitoring a process variable of a medium (5) in a container (4),
comprising
- a crystal body (6) with at least one defect (7),
- a magnetic field device (8) for generating a magnetic field,
wherein the magnetic field device (8) is arranged in such a way that a magnetic field (B) can be generated by means of the magnetic field device (8) in the region of the crystal body (6) and in the region of the medium (5) located inside the container (4), and
wherein the crystal body (6) and the magnetic field device (8) can be arranged from the outside on a wall of the container (4),
- a detection unit (9) for detecting a magnetic field-dependent fluorescence signal (2) from the crystal body (6),
wherein the detection unit (9) has an excitation unit (10) for optical excitation of the defect (7) and a detector (11) for detecting the fluorescence signal (2),
and
- an evaluation unit (12) for determining at least one statement about the process variable on the basis of the fluorescence signal (2).

2. Sensor device (3) according to claim 1,
wherein the magnetic field device (8) comprises at least one coil, and
wherein the coil at least partially surrounds the crystal body (6) when the crystal body (6) and the magnetic field device (8) are arranged on the wall of the container (4).

3. Sensor device (3) according to claim 1 or 2,
comprising an optical fiber (14) for conducting the fluorescence signal (2) from the crystal body (6) to the detection unit (9).

4. The sensor device (3) according to any one of claims 1 to 3,
comprising an enclosure (13) for inserting at least one component (6) of the sensor device (3) into a wall of the container (4).

5. Method for determining and/or monitoring a process variable of a medium (5) in a container (4) by means of a sensor device (3) according to one of the preceding claims 1 to 4,
comprising the following method steps:
- Generation of a magnetic field (B) in the region of the crystal body (6) and in the region of the medium (5) located inside the container (4),
- Excitation of the defect (7) in the crystal body (6) to fluorescence,
- detecting the magnetic field-dependent fluorescence signal (2) from the crystal body (6), and
- Determining at least one statement about the process variable using the fluorescence signal (2).

6. Method according to claim 5,
wherein at least one variable characteristic of the magnetic field (B), in particular the magnetic susceptibility or the magnetic permeability, is determined on the basis of the fluorescence signal (2).

7. Method according to claim 6,
wherein at least one physical and/or chemical parameter of the medium (5) is determined on the basis of the variable characteristic of the magnetic field (B).

8. Method according to claim 6 or 7,
whereby a status monitoring of a process taking place in the container (4) is carried out on the basis of the variable characteristic of the magnetic field (B).

9. The method according to any one of claims 6 to 8,
wherein a predeterminable limit level of the medium (5) in the container (4) is monitored, wherein a limit value for the quantity characteristic of the magnetic field (B) is predetermined, and
wherein in the event that the limit value predetermined for the quantity characteristic of the magnetic field (B) is exceeded or undershot, the medium (5) is signaled to reach the predeterminable limit level.

10. The method according to any one of claims 6 to 9,
wherein the magnetic field (B) generated in the region of the crystal body (6) and in the region of the medium (5) located within the container (4) is generated in the form of an alternating field, and
wherein the frequency of the alternating field (B) is varied.

## Revendications

1. Dispositif de détection (3) pour déterminer et/ou surveiller une grandeur de processus d'un milieu (5) dans un récipient (4),
comprenant
- un corps cristallin (6) présentant au moins un défaut (7),
- un dispositif de champ magnétique (8) pour générer un champ magnétique,
le dispositif de champ magnétique (8) étant disposé de telle sorte qu'un champ magnétique (B) puisse être généré au moyen du dispositif de champ magnétique (8) dans la zone du corps cristallin (6) et dans la zone du milieu (5) se trouvant à l'intérieur du récipient (4), et
le corps cristallin (6) et le dispositif de champ magnétique (8) pouvant être disposés de l'extérieur sur une paroi du récipient (4),
- une unité de détection (9) pour détecter un signal de fluorescence (2) dépendant du champ magnétique provenant du corps cristallin (6),
l'unité de détection (9) présentant une unité d'excitation (10) pour l'excitation optique du défaut (7) et un détecteur (11) pour la détection du signal de fluorescence (2),
et
- une unité d'évaluation (12) pour déterminer au moins une information sur la grandeur de processus à l'aide du signal de fluorescence (2).

2. Dispositif de détection (3) selon la revendication 1,
dans lequel le dispositif de champ magnétique (8) comprend au moins une bobine, et dans lequel la bobine entoure au moins partiellement le corps cristallin (6) lorsque le corps cristallin (6) et le dispositif de champ magnétique (8) sont disposés sur la paroi du récipient (4).

3. Dispositif de détection (3) selon la revendication 1 ou 2,
comprenant une fibre optique (14) pour conduire le signal de fluorescence (2) du corps cristallin (6) à l'unité de détection (9).

4. Dispositif de détection (3) selon l'une des revendications 1 à 3,
comprenant une bordure (13) pour l'insertion d'au moins un composant (6) du dispositif de détection (3) dans une paroi du récipient (4).

5. Procédé de détermination et/ou de surveillance d'une grandeur de processus d'un fluide (5) dans un récipient (4) au moyen d'un dispositif de détection (3) selon l'une des revendications précédentes 1 à 4,
comprenant les étapes de procédé suivantes :
- la génération d'un champ magnétique (B) dans la zone du corps cristallin (6) et dans la zone du milieu (5) se trouvant à l'intérieur du récipient (4),
- exciter le défaut (7) dans le corps cristallin (6) pour qu'il devienne fluorescent,
- détecter le signal de fluorescence dépendant du champ magnétique (2) provenant du corps cristallin (6), et
- détermination de la au moins une information sur la grandeur de processus à l'aide du signal de fluorescence (2).

6. Procédé selon la revendication 5,
au moins une grandeur caractéristique du champ magnétique (B), notamment la susceptibilité magnétique ou la perméabilité magnétique, étant déterminée à l'aide du signal de fluorescence (2).

7. Procédé selon la revendication 6,
au moins une grandeur caractéristique physique et/ou chimique du milieu (5) étant déterminée à l'aide de la grandeur caractéristique du champ magnétique (B).

8. Procédé selon la revendication 6 ou 7,
une surveillance de l'état d'un processus se déroulant dans le récipient (4) étant effectuée à l'aide de la grandeur caractéristique du champ magnétique (B).

9. Procédé selon l'une quelconque des revendications 6 à 8,
un niveau limite prédéfinissable du fluide (5) dans le récipient (4) étant surveillé,
une valeur limite étant prédéfinie pour la grandeur caractéristique du champ magnétique (B), et
dans le cas où la valeur limite prédéfinie pour la grandeur caractéristique du champ magnétique (B) est dépassée vers le haut ou vers le bas, on signale que le niveau limite prédéfinissable du milieu (5) est atteint.

10. Procédé selon l'une quelconque des revendications 6 à 9,
le champ magnétique (B) généré dans la zone du corps cristallin (6) et dans la zone du milieu (5) se trouvant à l'intérieur du récipient (4) étant généré sous la forme d'un champ alternatif, et
la fréquence du champ alternatif (B) étant modifiée.
